# EUROPEAN PATENT SPECIFICATION

(11) **EP 4 252 758 B1**
(45) Date of publication and mention of the grant of the patent: **10.12.2025**
(21) Application number: 21909329.1
(22) Date of filing: 20.12.2021
(51) Int. Cl.: A61K 33/24, A61P 29/00, A61P 25/04, A61P 25/06, A61K 31/55, A61K 31/24, A61K 31/28

(54) **TREATING PAIN BY VANADIUM COMPOUND**
SCHMERZBEHANDLUNG MIT EINER VANADIUMVERBINDUNG
TRAITEMENT DE LA DOULEUR À L'AIDE D'UN COMPOSÉ DE VANADIUM

(30) Priority: 21.12.2020 CN 202011514811
(43) Date of publication of application: 04.10.2023
(73) Proprietor: Shenzhen Fangshengtai Medical Technology Co., Ltd., Shenzhen, Guangdong 518122 (CN)
(72) Inventor: XIE, Fang, Changsha, Hunan 410205 (CN)
(74) Representative: Murgitroyd & Company
(86) International application number: PCT/CN2021/139621
(87) International publication number: WO 2022/135333

(56) References cited:
- WO-A1-2020/259447
- WO-A2-97/47296
- CN-A- 102 309 507
- CN-A- 102 309 507
- CN-A- 102 309 509
- CN-A- 102 309 509
- CN-A- 112 121 041
- LI SHUAI, ET AL.: "Effects of Non-specific Inhibition of Spinal Protein Tyrosine Phosphatases on Inflammatory Pain and Its Underlying Mechanisms", ZHONGGUO YAOLIXUE TONGBAO - CHINESE PHARMACOLOGICAL BULLETIN, LINCHUANG YAOLI YANJIUSUO, HEFEI, CN, vol. 27, no. 10, 31 October 2011 (2011-10-31), CN , pages 1397 - 1400, XP055944689, ISSN: 1001-1978, DOI: 10.3969/j.issn.1001-1978.2011.10.015
- DANG YAQIONG, ET AL.: "Bioactivity, Molecular Mechanism and Drug Discovery of Vanadium Complexes", PROGRESS IN PHARMACEUTICAL SCIENCES, CHINA PHARMACEUTICAL UNIVERSITY, CN, vol. 44, no. 4, 30 April 2020 (2020-04-30), CN , pages 256 - 268, XP055944692, ISSN: 1001-5094

## Description

### TECHNICAL FIELD

The present invention relates to a pharmaceutical composition for treating pain, particularly acute pain, chronic pain, inflammatory pain, cancerous pain, pain resulting from cancer therapy, visceral pain, neuropathic pain, pain resulting from diabetic neuropathy, post-herpetic pain, migraine, fibromyalgia, and trigeminal neuralgia.

### BACKGROUND

Pain is an unpleasant subjective sensation or emotional experience associated with tissue damage or potential tissue damage. Pain affects the normal life of individuals, families, and communities. According to a statistical study, 37.3% of adults in developed countries and 41.1% of adults in developing countries suffer from chronic pain. (Tsang, A., Von Korff, M., Lee, S., Alonso, J., Karam, E., Angermeyer, M. C., et al. (2008). Common chronic pain conditions in developed and developing countries: Gender and age differences and comorbidity with depression-anxiety disorders, Journal of Pain, 9 (10), 883 - 891.) Chronic pain may be associated with 1) limited mobility and daily activities (A. Gureje 0, Von Korff M, Simon GE, Gater R. Persistent pain and wellbeing. A World Health Organization study in primary care. JAMA 1998; 280:147 - 51. B. Smith BH, Elliott AM, Chambers WA, Smith WC, Hannaford PC, Penny K. The impact of chronic pain in the community. Fam Pract 2001; 18:292 - 9.), 2) addiction to opiates (Institute of Medicine. Relieving pain in America: a blueprint for transforming prevention, care, education, and research. Washington, DC: National Academies Press; 2011.), 3) anxiety and depression (A.), and 4) poor perceived health status and decreased quality of life (A and B). In the United States, approximately 50 million adults are affected by pain, 20 million of whom are seriously affected and cannot work or live normally most of the time (even every day). (Schappert SM, Burt CW. Ambulatory care visits to physician offices, hospital outpatient departments, and emergency departments: United States, 2001 - 02. Vital Health Stat 13 2006; 13:1-66.)

The mechanism of algogenesis is extremely complex. Although pain can be confirmed by electrophysiological methods, it is after all a subjective feeling. The intensity and quality of pain are related to both internal and external factors, and the same stimulus may give different experiences in different environments and at different physical and psychological states.

The nervous system recognizes and interprets various types of thermal and mechanical stimulation, as well as those brought by environmental and endogenous chemical stimuli. Clinically, acute and chronic pains are quite different. Acute pain, which is associated with skeletal muscle spasm and sympathetic nervous system activation, is caused by a specific disease or injury, serves a useful biological purpose, and is self-limited. In the case of continuous injury, both the peripheral and central nervous systems (i.e. constituent parts of the pain transmission pathway) exhibit tremendous plasticity, and at this point, pain signals are enhanced and allergic reactions occur as well. This may be beneficial when the plasticity facilitates defense reflex, but may lead to chronic pain when the changes persist. In contrast, chronic pain can be considered a disease state. It is pain that outlasts the normal time of healing, if associated with disease or physical injury. Chronic pain may arise from psychological states, serves no biological purpose and has no recognizable endpoint. Persistent pain associated with trauma or disease (such as diabetes, arthritis or tumor growth) may possibly be induced by peripheral neuropathic changes. Such changes can be caused by damaging nerve fibers and may lead to an increase of spontaneous discharge or changes in the conductivity or neurotransmitter properties. In fact, the utility of the systemic application of local anesthetics in the treatment of different neuropathic pain (e.g. post-herpetic neuralgia) may reflect their effect on sodium channels accumulated in damaged nerve fibers.

With the continuous understanding and study of pain, many analgesic drugs have been developed one after another. These drugs can be categorized as follows: 1) non-steroidal anti-inflammatory drugs: such as aspirin, ibuprofen, naproxen, ketoprofen, diclofenac, etc., and COX-2 inhibitors (such as rofecoxib, valdecoxib, etc.); 2) opiate analgesics: such as morphine, oxycodone, buprenorphine, fentanyl, etc.; 3) analgesics and antipyretics: such as acetaminophen, etc.; and antidepressants: such as nortriptyline, desipramine, etc.; 4) anticonvulsants: such as carbamazepine, gabapentin, etc.; 5) antipsychotic drugs: such as olanzapine, quetiapine, etc.; 6) serotonin receptor agonists: such as sumatriptan, ergotamine, lasmiditan, etc.; 7) CGRP (calcitonin gene-related peptide) inhibitors: such as erenumab, fremanezumab, etc.; and 8) other types of analgesics: such as ziconotide, etc.

Most non-steroidal anti-inflammatory drugs and opiate analgesics are broad-spectrum analgesics, i.e. drugs that can be used for a variety of pain indications, while the other categories of analgesics have significant limitations. As far as a single analgesic drug is concerned, each drug has obvious defects in one or more aspects such as efficacy, drug resistance and side effects. For example, non-steroidal anti-inflammatory drugs are only effective for mild and moderate pain; the most commonly used aspirin and ibuprofen may have severe side effects in the gastrointestinal tract; and opiate drugs have the problem of drug resistance, along with possible side effects such as constipation, respiratory depression, addiction, etc. Therefore, it is necessary to find better analgesic drugs.

Vanadium belongs to the group VB in the periodic table, and is a trace element necessary for the human body. Vanadium can exist in a variety of oxidation states in nature, and its oxidation states in organisms are +3, +4, and +5. Vanadium in the +3 oxidation state may exist as V³⁺ ions in the cells of a few lower marine organisms (Crans DC, Mahroof-Tahir M, Keramidas AD. Mol Cell Biochem, 1995, 153: 17-24), whereas under physiological conditions in animals, vanadium exists mainly in the +4 and +5 oxidation states.

Over the years, the medicinal value of vanadium compounds has been continuously explored. For example, it has been found that vanadium compounds have the activity of inhibiting protein tyrosine phosphatase 1B (PTB1B), can induce Hsp60-PPARγ protein interaction to eliminate the insulin resistance of tissues, and in the meantime upregulate the adiponectin level to activate PPARα. Therefore, vanadium compounds have the ability to increase the level of cellular energy metabolism and improve glucose/lipid metabolism, and are expected to become drugs for the treatment of diabetes and Alzheimer's Disease (AD). Vanadium compounds also have antiproliferative activity, thus can be potentially used in antitumor or anticancer therapy. In addition, vanadium compounds were found to have antiparasitic, antibacterial and antiviral effects. (1. Rehder, D. Perspectives for vanadium in health issues. Future Med. Chem. xxx; 2. Literature cited in US6,232,340 B; 3. Yaqiong Dong, Xia Niu, Ruyue Xiao, Zhang Yue, Qing Xia, Xiaoda Yang, The pharmacological/toxicological effects and rational drug design of vanadium complexes SCIENTIA SINICA Chimica, 2017, 47 (2), 162-171).

In his patents (CN102309507A and CN102309509A), Lei proposed the analgesic use of an inorganic salt mixture of a small amount of a vanadium salt in the +5 oxidation state with a vanadium salt in the +3 oxidation state and/or a vanadium salt in the +4 oxidation state, but the extremely acidic solution used to soak feet had severe side effects, giving poor patient compliance.

In another case, in the study of the mechanism of action of spinal cord protein tyrosine phosphatases (PTPs) in inflammatory pain, Li et al. intrathecally injected sodium orthovanadate (a phosphatase inhibitor) into the mice that suffered from pain due to induced inflammation, and found the analgesic effect, so the relationship between the activity of spinal cord protein tyrosine phosphatases and inflammatory pain was proposed. However, not only did the study not trigger any research on using vanadium compounds as analgesic drugs, but it has led to the belief that vanadium compounds can hardly become analgesic drugs administered in other ways than intrathecal injection. Phosphatases are widely present in the human body, such as blood, cell tissue, and bones, and if vanadium compounds are administered orally, by blood injection, subcutaneously, transdermally, etc., vanadium-containing ions will very likely bind to the phosphatases along the "pathway" (e.g. in blood) before reaching the spinal cord. This raises two questions: a) Can vanadium ions be transported into the spinal cord and bind to the phosphatases therein? b) Can the overall physiological response be analgesic after binding to so many phosphatases in the "pathway"? For such reasons, no report has been found on a subsequent study of the analgesic use of vanadium compounds from either Li's group or other research groups.

In the clinical study of treating diabetes and other diseases by using compounds containing vanadium, the therapeutic doses of such compounds were found to be an issue in bringing serious side effects, hurdling them to become analgesic drugs. The inventor unexpectedly found that compounds containing vanadium could effectively treat pain at a small dose, and that when the dose was increased to a certain level, the level of pain treatment reached a plateau. Therefore, a certain dose of vanadium compounds can not only treat pain, but also avoid the side effects of the vanadium-containing compounds. Thus, the present invention is achieved.

### SUMMARY

Features of the present invention are set forth in the appended Claims.

The present invention provides a positive-tetravalent vanadium compound or a positive-pentavalent vanadium compound comprising a vanadium-oxygen bond for use in treating pain, wherein the positive-tetravalent vanadium compound or the positive-pentavalent vanadium compound is used in an amount of 0.00001 mg/kg body weight to 300 mg/kg body weight (preferably 0.0001 mg/kg body weight to 50 mg/kg body weight, more preferably 0.001mg/kg body weight to 5mg/kg body weight) per day, based on the mass of vanadium, and the compound is prepared into an injection administered subcutaneously, intramuscularly or intravenously, with the proviso that the positive-tetravalent vanadium compound is not a metal complex formed by VO(IV) and a ligand of alpha-hydroxypyranone or alpha-hydroxypyridone type.

The vanadium compound of the present invention can be an inorganic vanadium compound and an organic vanadium compound. The inventor found that no matter whether a vanadium-containing compound is an organic compound or an inorganic compound and whether the valence state of vanadium contained is tetravalent or pentavalent, it can generate an analgesic effect as long as it is absorbed by a mammal (or a human body), and differences in the valence state or ligands thereof can affect onset time, analgesic intensity and duration. Under the physiological conditions of the human body, vanadium mainly exists in the form of tetravalent or pentavalent, and in the form of oxovanadium, so the active substances which exert the analgesic effect *in vivo* should be those containing V^{IV}O and V^{V}O moiety. Specifically, the vanadium compound of the present invention encompasses: a) a positive-pentavalent vanadium compound, i.e. a compound prepared by forming chemical bond(s) [i.e. ionic bond(s) and/or covalent bond(s) and secondary bond(s)] between positive-pentavalent vanadium [also represented by "pentavalent vanadium", "+5-valent vanadium", "V^{V}", "V⁵⁺", "vanadium (V)", or "V(V)"] and other atom(s) [or ion(s)]; and b) a positive-tetravalent vanadium compound, i.e. a compound prepared by forming chemical bond(s) [i.e. ionic bond(s) and/or covalent bond(s) and secondary bond(s)] between positive-tetravalent vanadium [also represented by "tetravalent vanadium", "+4-valent vanadium", "V^{IV}", "V⁴⁺", "vanadium (IV)", or "V(IV)"] and other atom(s) [or ion(s)]. The secondary bond comprises dipole-dipole interaction, London dispersion interaction, hydrogen bond, etc.

Preferably, the vanadium (V) or vanadium (IV) compound is a vanadium compound containing a vanadium-oxygen bond, a vanadium-halogen bond, a vanadium-nitrogen bond, a vanadium-phosphorus bond, a vanadium-sulfur bond or a vanadium-carbon bond or containing two or more of the above bonds. The vanadium compound often exists in the form of a complex, characterized in that: a) the ligand can be an inorganic ligand or an organic ligand; b) the ligand can be a neutral molecule, a negatively charged ion, or a positively charged ion; c) the ligand can be a monodentate (containing one donor) or polydentate (containing two or more donors, generally O-, N-, S- or C-donors) ligand; d) the complex can contain only one monodentate ligand, two or more monodentate ligands, only one polydentate ligand, two or more polydentate ligands, or both monodentate and polydentate ligands; e) the vanadium (V) or vanadium (IV) compound can be a covalent compound containing only covalent bond(s) [comprising coordination bond(s)] or a salt compound containing only ionic bond(s) or containing both ionic and covalent bonds; and the vanadium (V)- or vanadium (IV)-containing moiety can be present in the anion(s) or cation(s) of a salt; f) the vanadium (V) or vanadium (IV) compound can exist in a mononuclear or polynuclear polymeric form, wherein "nucleus" refers to a vanadium (V)- or vanadium (IV)-containing center; and g) the vanadium (V) or vanadium (IV) compound, in the solid state, is often accompanied by a crystal solvent such as water, methanol, ethanol, isopropanol, pyridine, DMSO, etc.

In general, the coordination number (CN) of a vanadium coordination compound varies from 4 to 8, and the geometric shape formed by coordination is: tetrahedron, square plane, triangular bipyramid, square pyramid, octahedron, triangular prism, pentagonal bipyramid, capped octahedron, capped triangular prism, square antiprism, dodecahedron. The vanadium compound actually formed may somewhat deviate from those summarized above in terms of coordination number or geometric shape.

The vanadium (V) or vanadium (IV) compound can have different morphologies in solid state or in a solution, and the structural features described in the present patent are those contained or partially contained in the solid state or solution state.

More preferred vanadium compound is a vanadium (V) or vanadium (IV) compound containing a vanadium-oxygen bond, including, but not limited to, the following types of compounds:
I. oxides of vanadium (V), including, but not limited to, V₂O₅ and hydrates thereof (such as V₂O₅·H₂O, V₂O₅·2H₂O, V₂O₅·8H₂O, etc.); oxides of vanadium (IV), including, but not limited to, VO₂, V₂O₄ and hydrates thereof;
II. vanadic (V) acids, including, but not limited to, orthovanadic acid (H₃VO₄), metavanadic acid (HVO₃), polyvanadic acids (such as H₄V₂O₇, H₄V₄O₁₂, H₅V₅O₁₅, H₆V₁₀O₂₈, H₃V₃O₉, H₄V₄O₁₂), heteropolyacids of vanadium (such as vanadophosphoric heteropolyacid, PPV, PVP, vanadoarsenic heteropolyacid), etc.; vanadic (IV) acids, including, but not limited to, H₄VO₄, H₆VO₅, H₂VO₃, polyacids (such as H₂V₂O₅), heteropolyacids (such as vanadophosphoric heteropolyacid, e.g. H₂[VO(P₂O₇)], vanadoarsenic heteropolyacid, vanadomolybdenic heteropolyacid, etc.), etc.;
III. salts formed between various vanadate anions and inorganic or organic cations,
   wherein the vanadate (V) anions include, but are not limited to: orthovanadates (i.e., VO₄³⁻, and protonated forms thereof, VO₄H₂⁻ and VO₄H²⁻ ), metavanadate (i.e.VO³⁻), polyvanadates (such as V₂O₇⁴⁻, V₄O₁₂⁴⁻, V₅O₁₅⁵⁻*,* V₁₀O₂₈⁶⁻, V₃O₉³⁻, V₄O₁₂⁴⁻, etc., and
   protonated forms thereof), heteropolyates (such as phosphorus-vanadium heteropolyate 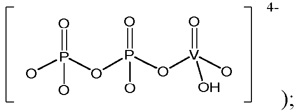 vanadate (IV) anions such as VO₄⁴⁻ (and protonated forms thereof, HₙVO₄⁴⁻ⁿ, n = 1-3 ), VO₅⁶⁻ (and protonated forms thereof, HₙVO₄⁶⁻, n = 1-5), VO₃²⁻ (and protonated form thereof, HVO₃⁻ ), V₂O₅²⁻ (and protonated form thereof, HV₂O₅⁻), V₄O₉²⁻ (and protonated form thereof, HV₄O₉⁻), and heteropolyates (such as VO(P₂O₇)²⁻, M_{O3}V₃O₁₉⁸⁻);
   wherein the inorganic or organic cations include, but are not limited to: quaternary ammonium ions, i.e. R₁R₂R₃R₄N⁺ [R₁, R₂, R₃ and R₄ can be H or lower alkane, or at least one pair of R₁ to Rₐ can be linked (via intervening atom(s) to form a substituted or unsubstituted nitrogen-containing heterocyclic ring, for example, R₁R₂R₃R₄N⁺ may represent NH₄⁺ or ammonium, PyH⁺ or pyridinium, an *N*-alkylpyridinium, etc.]; alkali metal cations, such as Na⁺, K⁺, Li⁺, Rb⁺; alkaline earth metal cations, such as Mg²⁺, Ca²⁺, Be²⁺, Sr²⁺, Ba²⁺, etc.; metal cations from Groups IIIA, IVA, VA and VIA; and transition metal cations, such as Fe²⁺, Fe³⁺, Cu²⁺, Zn²⁺, Ti²⁺, Ti³⁺, Ti⁴⁺, Ni²⁺, Ni³⁺, Cr²⁺, Cr³⁺, and Cr⁶⁺;
   specific examples of vanadium (V) salts are NH₄VO₃, Ca(VO₃)₂·4H₂O, K₂Ca(VO₃)₄·7H₂O, Rb₄Ca₄(V₂O₇)₃·17H₂O, Na₃VO₄, KCaVO₄·6H₂O, Na₄V₂O₇, K₃V₅O₁₄, Ca₃V₁₀O₂₈, BiVO₄, FeVO₄, etc.; and specific examples of vanadium (IV) salts are M^{I}₄VO₄, M^{II}₃VO₅, M^{II}₂VO₄, M^{I}₂VO₃, M^{I}₂V₂O₅, and K₂V₃O₇·2.66H₂O, wherein M^{I} is a +1-valent metal ion and M^{II} is a +2-valent metal ion;
IV. oxovanadium compounds formed by complexation between vanadium and monodentate or polydentate ligands, including, but not limited to:
   a) oxovanadium compounds formed by complexation between vanadium and monodentate ligand(s),
      wherein the monodentate ligands include, but are not limited to: F⁻, Cl⁻, Br⁻, I⁻, NO, H₂O, CN⁻, NO₂⁻, SCN⁻, NCS⁻, NH₃, N₃⁻, OH⁻, CO, HCO₃⁻, H₂PO₄⁻, HSO₄⁻, HSO₃⁻, ClO₄⁻, NO₃⁻, SO₃F⁻, HCOO⁻, sulfhydryls (e.g. SH⁻), phosphates (e.g. diphosphate), and organic monodentate ligands containing an O-, S-, or N-donor including, but not limited to, alcohols, phenols, amines, carboxylates, and organophosphates.

Specifically, the vanadium (V) compound is, for example, VOF₃, VOCl₃, VOBr₃, VO(ClO₄)₃, VO(NO₃)₃, VOCl₂(N₃), VOHal₂(OR), VOHal(OR)₂, VO(OR)₃, M₃[VO₂F₄], M₂[VO₂F₃], M[VO₂Cl₂], M[VOF₄], M₂[VOF₅], M₂[VOCl₅], [PyH][VOCl₄], M₃VO₄, M₃VO₃S, M₃VO₂S₂, M₃VOS₃, M₄[O₃VSVO₃], VO(OOCR)₃, VO(NR₁R₂)₃, , 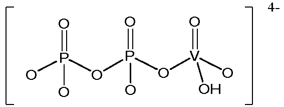 or the like, wherein M is Li, Na, K, Rb, or Cs; Hal = F, Cl, Br, or I; and R, R₁ and R₂ = H, alkane, aromatic hydrocarbon, or other organic groups.

Specifically, the vanadium (IV) compound is, for example, VOSO₄, 3VO₂(2SO₂), M^{I}₂[VO(SO₃)₂], M^{II}[VO(SO₃)₂], VOHal₂, VO(HalO₄)₂ (such as VO(ClO₄)₂·5H₂O), M^{I}₂[VOHal₄] or M^{I}₂[VOHal₅]{such as K₂[VOHal₄(H₂O)], Cs₃[VOCl₅]}, M^{I}₃[VO(CN)₅], M^{I}₂[VO(NCS)4], and Na(VO)₂(OH)₅, wherein Hal = F, Cl, Br, or I, M^{I} is a +1-valent metal ion, and M^{II} is a +2-valent metal ion;
b) oxovanadium compounds formed by complexation between vanadium and polydentate ligand(s),
wherein the polydentate ligands include, but are not limited to, inorganic ligands (such as CO₃²⁻, O²⁻, C₂O₄²⁻, SO₄²⁻, SO₃²⁻, PO₄³⁻, HPO₄²⁻, triphosphate, etc.) and polydentate organic ligands containing O-, N- or S-donors;
such as diols (such as ethylene glycol, saccharides, and nucleosides), glycerol, hydroxycarboxylic acids (such as α-hydroxycarboxylic acids such as lactic acid, citric acid, tartaric acid), salicylates, diacids (such as oxalic acid, malonic acid, succinic acid), hydroxamic acids (HONHCOR, R is an organic group), sulfur-containing ligands (such as β-mercaptoethanol, dithiothreitol, bis(2-mercaptoethyl)ether, tris(2-mercaptoethyl)amine, cysteine, glutathione, oxidized glutathione, disulfides, and related compounds or derivatives thereof), amino-alcohols and related ligands (such as ethanolamine, ethylenediamine, amino acids and derivatives thereof, 2-aminoethanethiol, diethanolamine and derivatives thereof, ethylene-*N,N*'-diacetic acid (EDDA) and analogs thereof, pyridine carboxylic acids, hydroxypyridine, amides), dipeptides, and polypeptides.

Specifically, the vanadium (V) compound is, for example, V₂O₃(SO₄)2·2H₂O, VOPO₄·2H₂O, M[VO₂(SO₄)], K₂[VO₂(C₂O₄)₂], M₃[VO₂(C₂O₄)₂], a diol ligand complex containing a 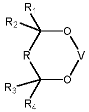 moiety, an amino-alcohol ligand complex containing a 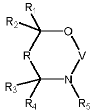 moiety, a hydroxycarboxylate ligand complex containing a 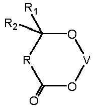 moiety, an amino acid ligand complex containing a 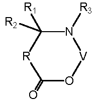 moiety, a 3-hydroxy-4H-pyran-4-one ligand complex containing a 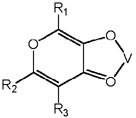 moiety, a hydroxamate ligand complex containing a 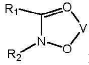 moiety, a diacid ligand complex containing a 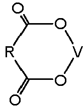 moiety, a sulfur-containing ligand complex containing a 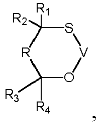 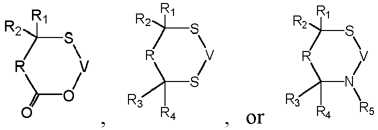 moiety, an α-pyridinecarboxylate ligand complex containing a 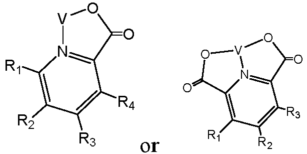 moiety, wherein, in the structural formula, V = V (V), M = Li, Na, K, Rb, or Cs; R₁, R₂, R₃, R₄ or R₅ is H, or other groups, and R can be a -(CH₂)ₙ- group, n = 0-4, -CR₁R₂-, or other groups such as homoatomic aromatic hydrocarbons, heterocyclic rings.

Specifically, the vanadium (IV) compound is, for example, V(SO₄)₂, VOSO₄, M^{II}₂[VO(SO₄)₃] or M^{I}₂[(VO)₂(SO₄)₃], (VO)₃(PO₄)₂, M^{II}[VO(PO₄)]₂ {such as Ba[VO(PO₄)]₂·4H₂O}, a diol ligand complex containing a 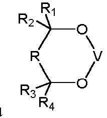 moiety, an amino-alcohol ligand complex containing a 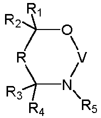 moiety, a hydroxycarboxylate ligand complex containing a 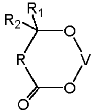 moiety, an amino acid ligand complex containing a 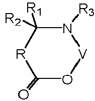 moiety, a 3-hydroxy-4*H-*pyran-4-one ligand complex containing a 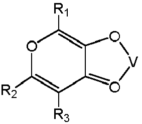 moiety, a hydroxamate ligand complex containing a 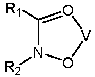 moiety, a diacid ligand complex containing a 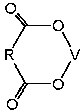 moiety, a sulfur-containing ligand complexes containing a 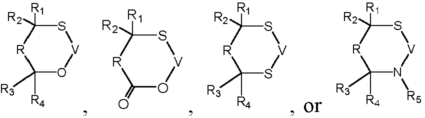 moiety, an α-pyridinecarboxylate ligand complex containing a 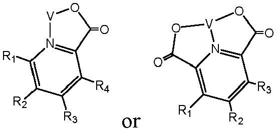 moiety, wherein M^{I} is a +1-valent metal ion, and M^{II} is a +2-valent metal ion; in the structural formula, V = V (IV), R₁, R₂, R₃, R₄ or R₅ is H, or other groups, and R can be a -(CH₂)ₙ-group, n = 0-4, -CR₁R₂-, or other groups such as homoatomic aromatic hydrocarbons, and heterocyclic rings.

The polydentate complexes are structurally characterized by their containing V-O, V-N and/or V-S bonds; vanadium and the ligands often form ring structures, such as a 5-membered ring and a 6-membered ring;
V. peroxovanadate compounds (peroxovanadates), i.e. compounds containing V(O₂) moiety, comprising, but not limited to, a) salts formed between the cations described in III and H₂VO₃(O₂)⁻, HVO₃(O₂)²⁻, VO₂(O₂)₂³⁻, HVO₂(O₂)₂²⁻, H₂VO₂(O₂)²⁻, HV(O₂)₃²⁻, and other peroxoranadate anions (for example, the structure of H₂VO₃(O₂)⁻ is 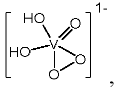 the structure of HVO₂(O₂)₂²⁻ is 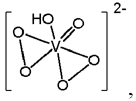 and the structure of HV(O₂)₃²⁻ is 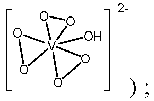 and b) compounds containing V(O₂) and complexed with the monodentate or polydentate ligands described in IV; wherein specific examples of the compounds are KH₂[VO₂(O₂)₂]·H₂O, (NH₄)₂H[VO₂(O₂)₂]·H₂O, etc.;
VI. hydroxamidovanadate compounds (hydroxamidovanadates), i.e. containing V (ONR₁R₂) moiety, wherein R₁ and R₂ are H or organic groups, comprising, but not limited to, a) salts formed between hydroxamidovanadate anions, such as VO₂(ONR₁R₂)₂⁻, having a structure of 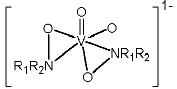 and the cations described in III; and b) compounds containing V(ONR₁R₂)₂ and complexed with the monodentate or polydentate ligands as described in IV; wherein R₁ and R₂ are H or other groups.

The vanadium compounds can exist in mononuclear, dinuclear and multinuclear forms. The vanadium compound formed by complexing a single ligand can be represented by the general formula VₐL_{b}, wherein V represents the vanadium nucleus (or vanadium center), L represents a ligand, and a and b represent quantities. The stoichiometric ratio of vanadium to the ligand can be 1/1, 1/2, 1/3, 2/3, 2/5, 3/2, 3/5, etc., and can be represented by the general formula a/b, wherein a or b is equal to an integer or decimal between 0 and 100. The vanadium compound formed by complexing two or more ligands can be represented by the general formula VₐL_{b}...Lⁿ⁻¹_{b(n-1)}, wherein b(n-1) is the quantity of Lⁿ, and a or b(n-1) is equal to an integer or decimal between 0 and 100. For example, if n = 1 (i.e. one ligand), the general formula is VₐL_{b}; if n = 2 (i.e. two ligands), the general formula is VₐL_{b}L¹_{b1} (i.e. containing two ligands: L and L¹; their quantities are b and b1 respectively); and if n = 3 (i.e. three ligands), the general formula is VₐL_{b}L¹_{b1},L²_{b2} (i.e. containing three ligands: L, L¹ and L²; their quantities are b, b1 and b2 respectively), and so on. In addition, the vanadium compounds can contain a varying quantity of solvent molecules in the solid state, such as H₂O, methanol, ethanol, isopropanol, pyridine, and DMSO.

Further preferred compounds: lithium orthovanadate, sodium orthovanadate, potassium orthovanadate, lithium polyvanadate, sodium polyvanadate, potassium polyvanadate, lithium metavanadate, sodium metavanadate, potassium metavanadate, the quaternary ammonium salt of metavanadate (i.e. R₁R₂R₃R₄N⁺VO₃⁻, R₁, R₂, R₃ and R₄ can be H, or other groups, lower alkanes, or at least one pair of R₁-R₄ can be linked (via intervening atom(s)) to form a substituted or unsubstituted nitrogen-containing heterocycle, for example, R₁R₂R₃R₄N⁺ can represent NH₄⁺ or ammonium, PyH⁺ or pyridinium, an N-alkylpyridinium, such as ammonium metavanadate, vanadyl sulfate (VOSO₄, or VOSO₄·nH₂O, n = 1-20), vanadyl dichloride, and compounds thereof containing crystal solvents (such as water, methanol, ethanol, isopropanol, DMSO, pyridine, DMF);
in addition, further preferred vanadium (V) or vanadium (IV) compounds comprise complexes formed between vanadium (V) or vanadium (IV) and the following ligands: a) hydroxycarboxylate compounds (including hydroxycarboxylic acids and hydroxycarboxylates), such as glycolic acid or glycolate, lactic acid or lactate, tartaric acid or tartarate, citric acid or citrate, α-hydroxyisobutyric acid or α-hydroxyisobutyrate, 2-ethyl-2-hydroxybutyric acid or 2-ethyl-2-hydroxybutyrate, malic acid or malate, mandelic acid or mandelate, and ascorbic acid or ascorbate; b) mono-, di- or poly-carboxylate compounds (mono-, di- or poly-carboxylic acids or carboxylates), such as formic acid or formate, acetic acid or acetate, stearic acid or stearate, oxalic acid or oxalate, malonic acid or malonate, alkyl- or aryl-malonic acid or malonate, sebacic acid or sebacate, succinic acid or succinate, phthalic acid or phthalate, etc.; c) mono-, di-, or poly-hydroxy compounds, such as methanol, ethanol, isopropanol, geraniol, menthol, retinol, and other mono-alcohols, compounds containing a 1,2- or 1,3-diol (such as ethylene glycol, 1,2-dihydroxypropane, 1,3-dihydroxypropane, 1,2-dihydroxycyclopentane, and 1,2-dihydroxycyclohexane), glycerol, mono-, di- or poly-saccharides and derivatives thereof, such as ribose, ADP, ATP, *D*-glucose, *D*-fructose, *D-*turanose, *D*-gluconic acid or *D*-gluconate, *L*-threonic acid or *L*-threonate, and amino- or acetamino-saccharides; c) phenols, diphenols or polyphenols, such as phenol, catechol or pyrocatechol, and polyhydroxy aromatic compounds; d) amino acids, dipeptides, polypeptides, protein and derivatives thereof (such as Schiff bases), such as cysteine, glutathione (GSH, gamma-L-glutamyl-L-cysteinyl-glycine) and GSMe (Me=methyl), GSSG, etc., *D*-aspartic acid, β-alanyl-L-histidine (carnosine), collagen, and serum proteins; amino acids (such as glycine, alanine, glutamic acid) and Schiff bases formed between the amino acids and salicylaldehyde or pyridoxal; e) diamines and Schiff bases formed therefrom, such as ethylenediamine, propylenediamine, phenylenediamine or other diamines, and Schiff bases formed between the diamines and salicylaldehyde or pyridoxal; f) β-diketones, such as acetylacetone, dibenzoylmethane, benzoylacetone, furoyltrifluoroacetone, and Schiff bases formed between the β-diketones and amines; g) hydroxypyrones and hydroxypyridinones such as maltol (3-hydroxy-2-methyl-4-pyrone), kojic acid (5-hydroxy-2-hydroxymethyl-4-pyrone), and ethylmaltol (2-ethyl-3-hydroxy-4-pyrone); h) phospholipids, including phosphatidylcholine (lecithin), phosphatidylethanolamine (cephalin), phosphatidylserine, phosphatidylinositol, phosphatidylglycerol or glycerol phospholipid, diphospholipid glycerol, sphingomyelin, etc.; i) hydroxylamines, such as N-hydroxylamines, *N,N-*dialkylhydroxylamines*,* 2- hydroxylamines, 3-hydroxylamines; j) ureas or biguanides; and k) a compound shown as formula I below: 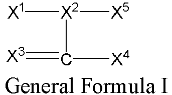
where X¹ and X³ are O, S, or NX⁶, preferably O or NX⁶;
X² is N or CX⁷;
X⁴, X⁵, X⁶ and X⁷ are non-labile protons or optionally substituted alkyl, aryl, aralkyl or alkaryl, or at least one pair of X⁴ to X⁷ (preferably X⁴ and X⁵) is linked [via intervening atom(s)] to form an optionally substituted saturated or unsaturated homocyclic (or carbocyclic) or heterocyclic ring, or where X¹ represents a NX⁶ group, X⁴ can represent an X⁸H group where X⁸ is oxygen or sulphur; and one proton to which X¹ or X⁸ is attached, preferrably X¹ attached proton, is labile.

Or a compound shown as formula II below:

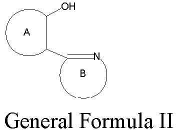

Another preferred category of ligands can be represented by general formula II, wherein A and B are 5-, 6- or 7-membered rings containing 0, 1 or 2 heteroatoms (selected from O, S, and N) in the ring and optionally substituted by X⁴, oxo, thio or NX⁴ (X⁴ is defined as above). Preferably, the A and B rings are 1,2-phenylene, oxazolin-2-yl, or thiazolin-2-yl, as shown below: 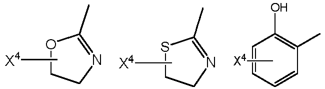 wherein X⁴ is H or an optionally substituted hydroxy C₁₋₄ alkyl group.

Preferred ligands corresponding to general formulas I and II include:
hydroxamates (general formulas III and IV) 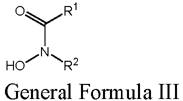 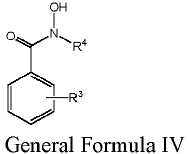
α-hydroxypyridinones (general formula V): 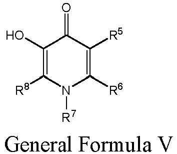
α-hydroxypyrones (general formula VI): 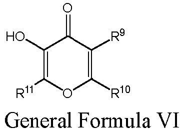
α-amino acids (general formula VII) 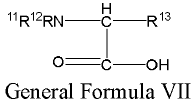
α-hydroxycarboxylic acids (general formula VIII) 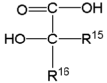
α-hydroxycarbonyl compounds (general formulas IX and X) 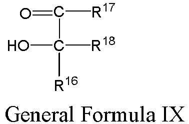 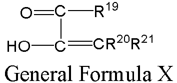
thiohydroxamates (general formulas XI and XII) 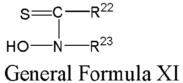 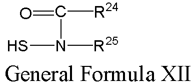
2-oxazolin-2-yl-phenols and 2-thiazolin-2-yl-phenols (general formulas XIII and XIV) 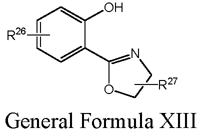 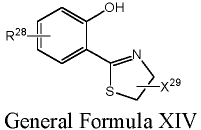
wherein R¹ to R²⁹ are H or any organic group such as any hydroxy C₁₋₄ alkane.

More preferred ligands are: 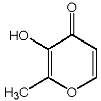
maltol 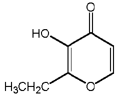
ethyl maltol 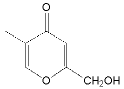
kojic acid
citric acid 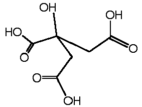
glycolic acid 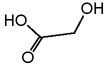
lactic acid *(L-,* D-or mixed-isomer) 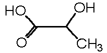
tartaric acid *(L-,* D-or mixed-isomer)

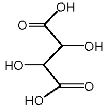

Complexes formed by vanadium (V)/vanadium (IV) and the aforementioned ligands can be synthesized by known methods (see USP 5, 620, 967, 4/1997, McNeill et al). The following compounds are particularly preferred according to the present invention: bis(maltolato)oxovanadium (BMOV), bis(ethylmaltolato)oxovanadium (BEOV), vanadyl kojate, vanadyl sulfate, sodium orthovanadate, potassium orthovanadate, sodium metavanadate, potassium metavanadate, and vanadium compounds prepared by mixing the aforementioned compounds with citric acid, tartaric acid, lactic acid, glycolic acid, ethylene glycol, glycerol, or triethanolamine [tris(2-hydroxyethyl)amine] at a molar ratio of 1:0.5 to 1:1000, and a citrate complex K[VO₂(C₆H₆O₇)]·H₂O.

The vanadium compounds of the present invention can be synthesized by conventional chemical synthesis techniques.

The vanadium compounds of the present invention can be used in combination with other analgesic drugs, such as forming complexes with other analgesic drugs, mixed with other analgesic drugs, or used with other analgesic drugs simultaneously or at intervals.

The compounds of the present invention can be prepared into various pharmaceutical compositions in conventional dosage forms, such as tablets, injections, capsules, patches, inhalants, etc.

These pharmaceutical compositions can be administered orally (e.g. prepared into tablets, coated tablets, dragees, hard or soft capsules, solutions, emulsions, or suspensions), rectally (e.g. prepared into suppositories), or parenterally (e.g. prepared into injections administered subcutaneously, intramuscularly or intravenously; e.g. prepared into patches or sprays administered transdermally; or e.g. prepared into inhalants administered through nasal or oral mucosa) .

Pharmaceutical compositions containing the compounds of the present invention can be prepared by a method known in the art, e.g. conventional mixing, encapsulation, dissolution, granulation, emulsification, enveloping, dragee making, or lyophilization. These pharmaceutical formulations can be prepared with therapeutically inert, inorganic or organic carriers. Lactose, corn starch or derivatives thereof, talc, stearic acids or salts thereof can be used as carriers for tablets, coated tablets, dragees, and hard gelatine capsules. Suitable carriers for the preparation of soft capsules comprise vegetable oils, waxes, and fatty oils. Suitable carriers for the preparation of solutions or syrups are water, polyols, sucrose, invert sugar, and glucose. Suitable carriers for injections are water, alcohols, polyols, glycerol, vegetable oil, phosphoric acid, and surfactants. Suitable carriers for suppositories are natural or hardened oils, waxes, fatty oils, and semi-solid polyols.

The pharmaceutical formulations can also contain preservatives, solubilizers, stabilizers or stabilizing agents, wetting agents, emulsifiers or emulsifying agents, sweeterners or sweetening agents, colorants or coloring agents, flavorants or flavoring agents, salts for changing the osmotic pressure, buffering agents, coating agents, or antioxidants.

A therapeutically effective amount of the compounds according to the present invention means an amount that is effective for preventing, alleviating or ameliorating the symptoms of a disease or prolonging the life of a patient being treated. The therapeutically effective amount or dosage of the compounds according to the present invention can vary wide limits. It is to be tailored to the individual conditions in each individual case which include the specific compound administered, the route of administration, the condition being treated, and the patient being treated. The metal complex is generally administered at a dosage of 0.00001 mg to 1500 mg (based on the mass of vanadium)/kg body weight per day depending on the subject to be administered, physical conditions, and the route of administration. First, the range of dosage is broad since the efficacy of a therapeutic effect for different mammals varies widely with doses typically being 10, 20, 30 or even more times smaller in man than in mice (unit body weight). Different routes of administration can also affect the dosage. For example, an oral dose can be 10 times the injection dose. The dosage range is preferably 0.00001 mg/kg body weight to 300 mg/kg body weight per day, more preferably 0.0001 mg/kg body weight to 50 mg/kg body weight per day, even more preferably 0.001 mg/kg body weight to 5 mg/kg body weight per day, based on the mass of vanadium.

The pharmaceutical unit dose is 0.001 mg to 1000 mg (based on metal atoms), preferably 0.01 mg to 300 mg (based on the mass of vanadium).

Generally, when orally administered to an adult of about 70 Kg, a daily dosage of about 0.0005 mg to about 500 mg (preferably about 0.005 mg to about 300 mg, more preferably about 0.05 mg to about 300 mg) (based on the mass of vanadium) should be appropriate; and for administration by injection, a daily dosage of about 0.001 mg to about 10 mg (preferably about 0.01 mg to about 5 mg) (based on the mass of vanadium) should be appropriate, although there are indications that the upper limit can be exceeded. The daily dose can be administered as a single dose or as divided doses.

Changes in the concentration and pH of vanadium (V) or vanadium (IV) in aqueous solutions can result in the formation of polymers, affecting the analgesic effect. The present invention provides a composition which, in addition to a vanadium (V) or vanadium (IV) compound, contains one or more of the following substances:
a) hydroxycarboxylate compounds (including hydroxycarboxylic acids and hydroxycarboxylates), such as glycolic acid or glycolate, lactic acid or lactate, tartaric acid or tartarate, citric acid or citrate, α-hydroxyisobutyric acid or α-hydroxyisobutyrate, 2-ethyl-2-hydroxybutyric acid or 2-ethyl-2-hydroxybutyrate, malic acid or malate, mandelic acid or mandelate, and ascorbic acid or ascorbate; b) mono-, di- or poly-carboxylate compounds (mono-, di- or poly-carboxylic acids or carboxylates), such as formic acid or formate, acetic acid or acetate, stearic acid or stearate, oxalic acid or oxalate, malonic acid or malonate, alkyl-or aryl-malonic acid or malonate, sebacic acid or sebacate, succinic acid or succinate, phthalic acid or phthalate; c) mono-, di-, or poly-hydroxy compounds, such as methanol, ethanol, isopropanol, geraniol, menthol, retinol, and other mono-alcohols, compounds containing a 1,2- or 1,3-diol (such as ethylene glycol, 1,2-dihydroxypropane, 1,3-dihydroxypropane, 1,2-dihydroxycyclopentane, and 1,2-dihydroxycyclohexane), glycerol, mono-, di- or poly-saccharides and derivatives thereof, such as ribose, ADP, ATP, D-glucose, D-fructose, D-turanose, D-gluconic acid or D-gluconate, L-threonic acid or L-threonate, and amino- or acetamino-saccharides; c) phenols, diphenols or polyphenols, such as phenol, catechol or pyrocatechol, and polyhydroxy aromatic compounds, d) amino acids, dipeptides, polypeptides, protein and derivatives thereof (such as Schiff bases), such as cysteine, glutathione (GSH, gamma-L-glutamyl-L-cysteinyl-glycine) and GSMe (Me=methyl), GSSG, etc., *D*-aspartic acid, β-alanyl-L-histidine (carnosine), collagen, and serum proteins; Schiff bases formed between amino acids (such as glycine, alanine, glutamica acid) and salicylaldehyde or pyridoxal; e) diamines and Schiff bases formed therefrom, such as ethylenediamine, propylenediamine, phenylenediamine or other diamines, and Schiff bases formed between the diamines and salicylaldehyde or pyridoxal; f) β-diketones, such as acetylacetone, dibenzoylmethane, benzoylacetone, furoyltrifluoroacetone, etc., and Schiff bases formed between the β-diketones and amine compounds; g) hydroxypyrones and hydroxypyridinones, such as maltol (3-hydroxy-2-methyl-4-pyrone), kojic acid (5-hydroxy-2-hydroxymethyl-4-pyrone), and ethylmaltol (2-ethyl-3-hydroxy-4-pyrone); h) phospholipids, including phosphatidylcholine (lecithin), phosphatidylethanolamine (cephalin), phosphatidylserine, phosphatidylinositol, phosphatidylglycerol or glycerol phospholipid, diphospholipid glycerol, sphingomyelin, etc.; i) hydroxylamine compounds, such as *N-* or *N,N*-alkylhydroxylamine, 2-hydroxylamine, 3-hydroxylamine, etc.; j) urea or biguanide compounds; and k) a compound shown as formula I below: 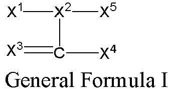
   X¹ and X³ are O, S, or NX⁶, preferably O or NX⁶;
   X² is N or CX⁷;
   X⁴, X⁵, X⁶ and X⁷ are non-labile protons or optionally substituted alkyl, aryl, aralkyl or alkaryl, or at least one pair of X⁴ to X⁷ (preferably X⁴ and X⁵) is linked [via intervening atom(s)] to form an optionally substituted saturated or unsaturated homocyclic (or carbocyclic) or heterocyclic ring, or where X¹ represents an NX⁶ group, X⁴ can represent an X⁸H group, wherein X⁸ is oxygen or sulphur; and one proton to which X¹ or X⁸ is attached, preferrably X¹ is labile.

Or a compound shown as formula II below:

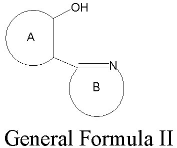

Another preferred category of ligands can be represented by general formula II, wherein A and B are 5-, 6- or 7-membered rings containing 0, 1 or 2 heteroatoms (selected from O, S, and N) in the ring and optionally substituted by X⁴, oxo, thio or NX⁴ (X⁴ is defined as above).

Preferably, the A and B rings are 1,2-phenylene, oxazolin-2-yl, or thiazolin-2-yl, as shown below: 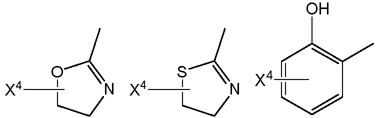 wherein X⁴ is H or an optionally substituted hydroxy C₁₋₄ alkyl group.

Preferred ligands corresponding to general formulas I and II include:
hydroxamates (general formulas III and IV) 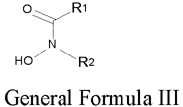 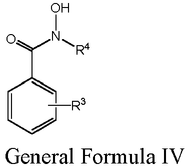
α-hydroxypyridinones (general formula V): 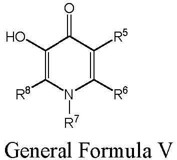
α-hydroxypyrones (general formula VI): 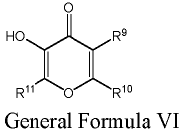
α-amino acids (general formula VII) 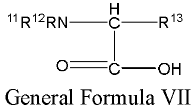
α-hydroxycarboxylic acids (general formula VIII) 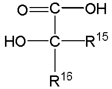
α-hydroxycarbonyl compounds (general formulas IX and X) 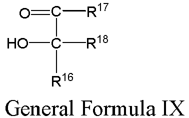 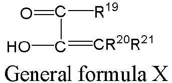
thiohydroxamates (general formulas XI and XII) 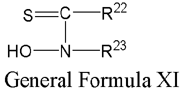 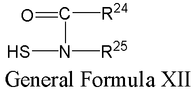
2-oxazolin-2-yl-phenols and 2-thiazolin-2-yl-phenols (general formulas XIII and XIV) 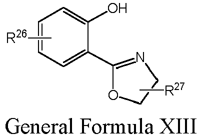 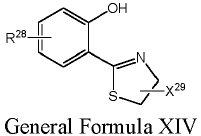
wherein R¹ to R²⁹ are H or any organic group such as any hydroxy C₁₋₄ alkane.

More preferred ligands are: 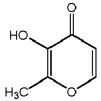
maltol 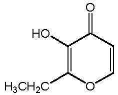
ethyl maltol 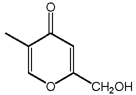
kojic acid
citric acid 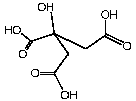
glycolic acid 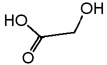
lactic acid *(L-,* D-or mixed-isomer) 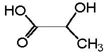
tartaric acid *(L-,* D-or mixed-isomer)

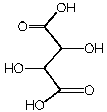

The weight ratio of the vanadium (V) compound to the aforementioned substances in the composition or the weight ratio of the vanadium (IV) compound to the aforementioned substances can be adjusted according to actual conditions to ensure that no polymer is formed by the vanadium (V) compound or the vanadium (IV) compound in the aqueous solution of the composition, so as to ensure analgesic efficacy. In this case, the pH value of the aforementioned aqueous solution should also be close to neutral, e.g. 6 to 8. For example, the vanadium (V) compound or the vanadium (IV) compound is mixed with citric acid, tartaric acid, lactic acid, glycolic acid, ethylene glycol, glycerol or triethanolamine (tris(2-hydroxyethyl)amine) in a molar ratio of 1:0.1 to 1:1000 to prepare the composition.

### BRIEF DESCRIPTION OF THE DRAWINGS

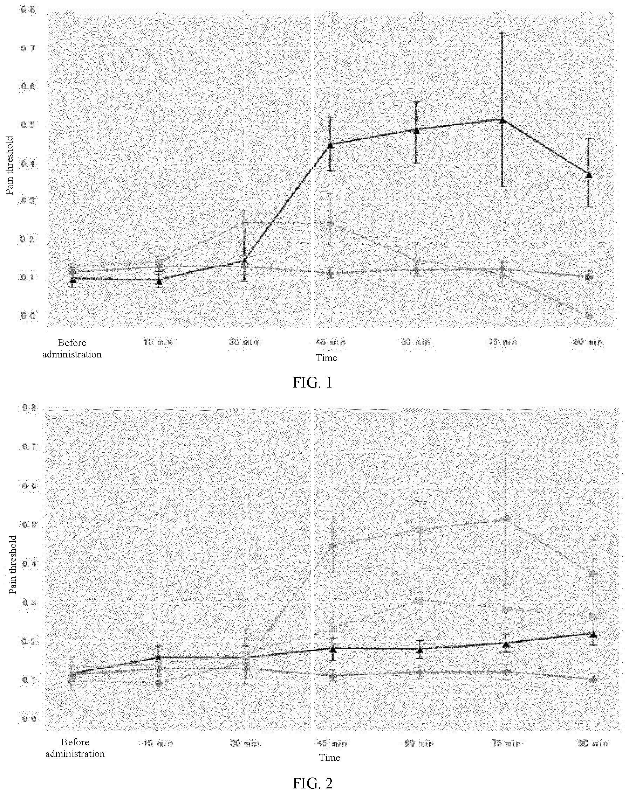

FIG. 1 is a graph of the effect of a sodium orthovanadate solution on the pain threshold of mice at a dose based on the atomic mass of vanadium.
   abscissa: time (unit: min or minute); ordinate: pain threshold;
   administration route: intraperitoneal injection;
   abscissa: time (unit: min or minute); ordinate: pain threshold (unit: g or gram);
   "▲": solution A1, administered at a dose of about 0.97 mg/kg;
   "●": aspirin solution (or solution S), administered at a dose of about 250 mg/kg;
   "+": blank control.
FIG. 2 is a graph of the effect of a sodium orthovanadate solution on the pain threshold of mice at different doses based on the atomic mass of vanadium.
   abscissa: time (unit: min or minute); ordinate: pain threshold (unit: g or gram);
   administration route: intraperitoneal injection;
   "●": solution A1, administered at a dose of about 0.97 mg/kg;
   "■": solution A2, administered at a dose of about 0.19 mg/kg;
   "▲": solution A3, administered at a dose of about 0.097 mg/kg;
   "+": blank control.
FIG. 4 is a graph of the effect of a sodium metavanadate solution on the pain threshold of mice at a dose based on the atomic mass of vanadium.
   administration route: intraperitoneal injection
   abscissa: time (unit: min or minute); ordinate: pain threshold (unit: g or gram);
   "▲": solution D, administered at a dose of about 0.97 mg/kg;
   "●": solution S, administered at a dose of about 250 mg/kg
   "+": blank control.
FIG. 5 is a graph of the effect of a sodium salt solution of an orthovanadate/citrate complex on the pain threshold of mice at a dose based on the atomic mass of vanadium.
   administration route: intraperitoneal injection
   abscissa: time (unit: min or minute); ordinate: pain threshold (unit: g or gram);
   "▲": solution B, administered at a dose of about 0.97 mg/kg;
   "●": solution S, administered at a dose of about 250 mg/kg;
   "+": blank control.
FIG. 6 shows the effect of a sodium salt solution of a metavanadate/citrate complex on the pain threshold of mice at a dose based on the atomic mass of vanadium.
   administration route: intraperitoneal injection
   abscissa: time (unit: min or minute); ordinate: pain threshold (unit: g or gram);
   "▲": solution E, administered at a dose of about 0.97 mg/kg;
   "●": solution ASA. administered at a dose of about 250 mg/kg;
   "+": blank control.
FIG. 7 shows the effect of a potassium salt solution of the metavanadate/citrate complex on the pain threshold of mice at a dose based on the atomic mass of vanadium.
   administration route: intraperitoneal injection
   abscissa: time (unit: min or minute); ordinate: pain threshold (unit: g or gram);
   "▲": solution F, administered at a dose of about 0.97 mg/kg;
   "●": solution ASA, administered at a dose of about 250 mg/kg;
   "+": blank control.

### DETAILED DESCRIPTION

In order to better understand the essence of the present invention, the usage of the compounds of the present invention in the treatment of pain will be illustrated below with the animal-experiment results of those compounds, from which it can be seen that the compounds of the present invention can have a rapid onset and long duration of action, producing a rapid and long-acting analgesic effect. Activity data of the compounds of the present invention are given in the activity-test examples.

### Example 1: Construction of Mouse Pain Model

10 µL of complete Freund's adjuvant (CFA) was injected into the left hind foot of mice (♂ Kunming mice, 22 g to 40 g) to induce inflammation. 24 hours after the injection, a Von Frey test was performed to determine a pain threshold. That is, under the guidance of Dixon's Up-Down method, Von Frey fibers were used to induce the mechanical paw withdrawal of the mice, and data were collected and calculated to obtain their 50% paw withdrawal thresholds (paw withdrawal threshold or PWT for short, i.e. pain threshold). Mice were then divided into different groups, including a normal saline group, a positive control group, and a drug group, which were intraperitoneally injected with normal saline, an aspirin (acetylsalicylic acid, or ASA) solution and a solution of compound for testing respectively. A Von Frey test was then performed to monitor changes in pain threshold.

After modeling, the pain thresholds of mice were reduced. An average threshold before the induction was about 2.5. Then, the mice with an average pain threshold of about 0.1 were selected. After the known analgesic aspirin was administered, the pain thresholds rose back to a certain degree, proving that the model was established successfully. Mice were divided into three groups: a blank control group, an aspirin (ASA) group, and a drug group. In blank control group, the mice were given normal saline, deionized water or deionized water containing ≤5% DMSO at the same volume as that in the drug group. In ASA group, the mice were given the known analgesic drug ASA as the positive control for tested compounds. In drug group, the mice were given compounds for testing accordingly. Due attention was paid to observe whether the pain threshold rose, the period during which the rise was maintained and the magnitude of the rise, after the injection of a tested compound solution. This way, the profiles of tested compounds were evaluated in terms of whether it has any analgesic effect, as well as its analgesic duration and intensity.

### Example 2: Preparation of a Positive Control Sample and Drug Samples for Testing

### 1. Preparation of a positive control

Solution S: Aspirin (ASA) was prepared into a solution of 0.11 M in tris-HCl solution by taking 0.1 M tris-HCl buffer solution as a solvent and adjusting the pH with a sodium hydroxide solution, until the final pH value lied approximately within the range between 7.4 to 7.6.

### 2. Preparation of sodium orthovanadate solution (solution A)

Sodium orthovanadate (Na₃VO₄): It was purchased from Sigma-Aldrich.
Solution A0: 184 mg of sodium orthovanadate was weighed and dissolved in 6 mL of deionized water, the pH value was adjusted to 10 by hydrochloric acid. The solution was heated until colorless, cooled to room temperature, and then the pH was detected. If the pH changed, the previous process was repeated until the pH was about 10. The solution was replenished with deionized water to 8 mL and stored at 4 °C, and this solution was called sodium orthovanadate stock solution.
Solution A1: The sodium orthovanadate stock solution (i.e. solution A0) was diluted with deionized water to prepare an aqueous sodium orthovanadate solution with a concentration of 1.5 × 10⁻³ M;
Solution A2: The solution A0 was diluted with deionized water to prepare an aqueous sodium orthovanadate solution with a concentration of 3.0 × 10⁻⁴ M;
Solution A3: The solution A0 was diluted with deionized water to prepare an aqueous sodium orthovanadate solution with a concentration of 1.5 × 10⁻⁴ M;
Solution A4: The solution A0 was diluted with deionized water to prepare an aqueous sodium orthovanadate solution with a concentration of 3.0 × 10⁻³ M;
Solution A5: The solution A0 was diluted with deionized water to prepare an aqueous sodium orthovanadate solution with a concentration of 7.5 × 10⁻⁴ M;
Solution A6: The solution A0 was diluted with deionized water to prepare an aqueous sodium orthovanadate solution with a concentration of 7.5 × 10⁻⁶ M;

### 3. Preparation of a sodium salt solution (solution B) of orthovanadate/citrate complex

Solution B: 14.8 mg of sodium orthovanadate was dissolved in 3.5 mL of water, and a 0.25 M citric acid solution was added dropwise thereto until the pH value of the mixed solution reached about 7. The mixed solution was replenished with a small amount of water to a total volume of 4 ml, giving a citrate complex solution of vanadium with a concentration of 0.020 M calculated according to vanadium. The solution was further diluted to a concentration of 1.5 × 10⁻³ M as a solution for testing.

### 5. Preparation of sodium metavanadate solution (or solution D)

Sodium metavanadate (NaVO₃): It was purchased from Sigma-Aldrich.

Solution D: Sodium metavanadate was prepared into an aqueous solution with a concentration of 3.0 × 10⁻³ M.

### 6. Preparation of sodium salt solution (solution E) of metavanadate/citrate complex

Solution E: 2 mL of a citric acid solution with a concentration of 0.135 M was added into 1 mL of a sodium metavanadate solution with a concentration of 0.26 M. The mixed solution was diluted to a concentration of 1.5 × 10⁻³ M as a solution for testing.

### 7. Preparation of potassium salt solution (solution F) of metavanadate/citrate complex

Preparation of potassium dioxo(citrato)vanadate (V) (i.e. hydrated potassium dioxo(citrato)vanadate (V) {K[VO₂(C₆H₆O₇)] · H₂O}): V₂O₅ (0.50 g, 2.75 mM) was suspended in an aqueous solution of KOH (0.31 g, 5.5 mM) and cooled in an ice bath, and citric acid solution (citric acid·H₂O: 1.21 g, 6.0 mM; water: 5 mL) was added dropwise with stirring. The mixed solution was left to stand for storage overnight at 4 °C (in a refrigerator), filtered and dried to give a pale white powder. The yield was 43%. (ref: Inorg. Chem. 1989, 719-723)

Solution F: 13 mg of solid K[VO₂(C₆H₆O₇)]·H₂O was dissolved in deionized water to prepare a solution with a concentration of 1.5 × 10⁻³ M as a solution for testing.

### 8. Preparation of vanadyl sulfate solution (solution G)

Vanadyl sulfate (VOSO₄ · 5H₂O): It was purchased from Alfa Aesar.

Solution G1: An aqueous vanadyl sulfate solution with a concentration of 1.5 × 10⁻³ M was prepared with deionized water.

Solution G2: An aqueous vanadyl sulfate solution with a concentration of 3.0 × 10⁻³ M was prepared with deionized water.

Solution G3: An aqueous vanadyl sulfate solution with a concentration of 7.5 × 10⁻⁴ M was prepared with deionized water.

Solution G4: An aqueous vanadyl sulfate solution with a concentration of 7.5 × 10⁻⁶ M was prepared with deionized water.

### 12. Preparation of bis(acetylacetonato)oxovanadium solution (solution K)

Bis(acetylacetonato)oxovanadium (IV) (i.e., vanadyl acetylacetonate or VO(acac)₂): It was purchased from Shanghai Qiming Biotechnology Co., Ltd.

Solution K: A bis(acetylacetonato)oxovanadium solution with a concentration of 1.5 × 10⁻³ M was prepared using DMSO and deionized water, with the content of DMSO being less than or equal to 5%.

### 13. Preparation of vanadyl oxalate solution (solution L)

Vanadyl oxalate: It was purchased from Shanghai Rongtai Pharmaceutical Technology Co., Ltd.

Solution L: A vanadyl oxalate solution with a concentration of 1.5 × 10⁻³ M was prepared using DMSO and deionized water, with the content of DMSO being less than or equal to 5%.

### 14. Preparation of bis(picolinato)oxovanadium solution (solution M)

Bis(picolinato)oxovanadium (IV) [i.e. VO(pic)₂ or BPOV]: It was purchased from Shanghai Qiming Biotechnology Co., Ltd.

Solution M: A bis(picolinato)oxovanadium solution with a concentration of 1.5 × 10⁻³ M was prepared with DMSO and deionized water, with the content of DMSO being less than or equal to 5%.

### 15. Preparation of sodium salt solution (solution N) of orthovanadate/ethylene glycol complex

Solution N: 1 mL of an aqueous ethylene glycol solution (1.8 × 10⁻² M) was added into 2 mL of solution A4 (concentration: 3.0 × 10⁻³ M), and deionized water was then added to the mixed solution to give a total volume of 4 mL.

### 16. Preparation of sodium salt solution (solution O) of orthovanadate/propylene glycol complex

Solution O: 1 mL of an aqueous 1,2-propanediol solution (1.8 × 10⁻² M) was added into 2 mL of solution A4 (concentration: 3.0 × 10⁻³ M), and deionized water was then added to the mixed solution to give a total volume of 4 mL.

### 17. Preparation of sodium salt solution (solution P) of orthovanadate/glycerol complex

Solution P: 1 mL of an aqueous 1,2-propanediol solution (1.8 × 10⁻² M) was added into 2 mL of solution A4 (concentration: 3.0 × 10⁻³ M), and deionized water was then added to the mixed solution to give a total volume of 4 mL.

### 18. Preparation of sodium salt solution (solution Q) of orthovanadate/lactate complex

A lactic acid solution (0.25 M) was added dropwise into 1 mL of a sodium orthovanadate solution (0.025 M) until the pH value of the mixed solution was about 7. The mixed solution was replenished with deionized water until the final concentration reached 1.5 × 10⁻³ M (calculated according to vanadium), giving a solution for testing.

### 19. Preparation of sodium salt solution (solution R) of orthovanadate/glycolate complex

A glycolic acid solution (0.25 M) was added dropwise into 1 mL of a sodium orthovanadate solution (0.025 M) until the pH value of the mixed solution was about 7. The mixed solution was replenished with deionized water to a concentration of 1.5 × 10⁻³ M (calculated according to vanadium), giving a solution for testing.

### Example 3: Analgesic Experiment of Sodium Orthovanadate Solution

A1 was intraperitoneally injected at the dose standard of 0.97 mg/kg (based on the atomic mass of vanadium), and collected data were compared with those of the ASA group and the blank group (FIG. 1).

| Tested Solution | Onset time (min) | Duration (min) |
|---|---|---|
| A1 | 30 to 45 after injection | >60 |
| S | 15 to 30 after injection | <45 |
| Blank | ineffective | null |

Description of the experimental results: The solution A1 began to affect the pain threshold of the mice 30 to 45 minutes after intraperitoneal injection, resulting in an increase in the pain threshold, and the pain threshold of the mice did not yet return to a baseline value 90 minutes after injection; the solution S began to boost the pain threshold 15 to 30 minutes after injection, but the pain threshold returned to the baseline value after about 60 minutes. For most of the time after the solution A1 began to boost the pain threshold, the thresholds of the mice in the A1 group were generally higher than those of the mice in the S group which were boosted by the solution S.

### Example 4: Analgesic Experiment of Sodium Orthovanadate Solutions at Different Concentrations

At the dose standards of 0.19 mg/kg and 0.097 mg/kg (based on the atomic mass of vanadium), A2 and A3 were intraperitoneally injected into two groups of mice, respectively, and collected data were compared with those of the A1 group and the blank group (FIG. 2).

| Tested Solution | Onset time (min) | Duration (min) |
|---|---|---|
| A1 | 30 to 45 after injection | >60 |
| A2 | 30 to 45 after injection | >60 |
| A3 | 30 to 45 after injection | >60 |
| Blank | ineffective | null |

Description of the experimental results: A1, A2 and A3 all began to boost the pain thresholds of the mice 30 to 45 minutes after injection, but the pain thresholds of the mice did not return to a baseline value 90 minutes after injection. However, as the administered concentration decreased, the magnitude of increase in the pain threshold decreased, i.e. A3<A2<A1.

As the summary of Examples 3 and 4, it can be concluded that: the sodium orthovanadate solutions have an analgesic effect; and not only can sodium orthovanadate exert an analgesic effect with intensities similar to or better than those of ASA at concentrations far lower than that of the ASA solution, but it has significantly longer duration of action than that of ASA. In addition, the examples have also shown the trend that the efficacy decreases with the decrease of sodium orthovanadate concentration, i.e. A3<A2<A1.

### Example 6: Analgesic Experiment of Sodium Metavanadate Solution

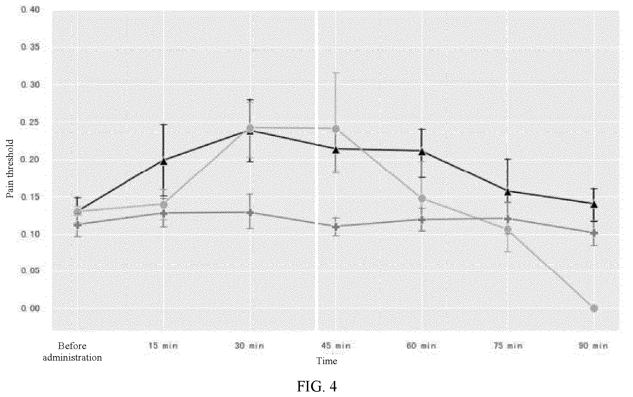

The solution D was intraperitoneally injected at the dose standard of 1.9 mg/kg, and collected data were compared with those of the ASA group and the blank group (FIG. 4).

| Tested Solution | Onset time (min) | Duration (min) |
|---|---|---|
| D | 0 to 15 after injection | >60 |
| S | 15 to 30 after injection | <45 |
| Blank | ineffective | null |

Description of the experimental results: The solution D began to affect the pain threshold of the mice 0 to 15 minutes after injection, resulting in an increase of the pain threshold, and the pain threshold of the mice approximated the baseline value 90 minutes after injection; the solution S began to boost the pain threshold 15 to 30 minutes after injection, but the pain threshold returned to the baseline value after about 60 minutes. For most of the time after the solution D began to affect the pain threshold, the thresholds of the mice in the D group were greater than or comparable to those of the mice, boosted by the solution S, in the S group .

Therefore, the sodium metavanadate solution has an analgesic effect. Not only can metavanadate exert analgesic effect with intensities similar to those of ASA at concentrations far lower than that of the ASA solution, but it has a significantly longer duration of action (>60 min) than that (<45 min) of ASA.

### Example 7: Analgesic Experiment of Sodium Salt Solution of Orthovanadate/Citrate Complex

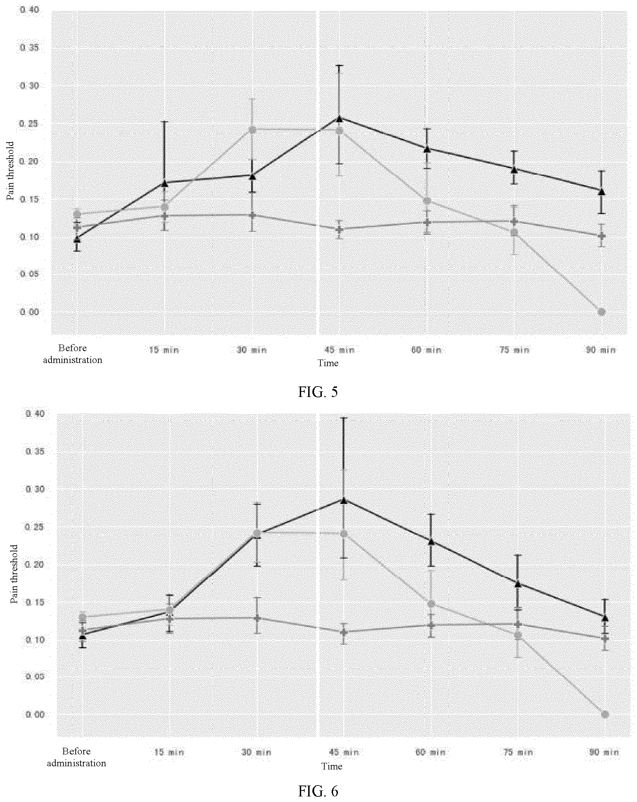

The solution B1 was injected at the dose standard of 0.97 mg/kg (based on the atomic mass of vanadium), and collected data were compared with those of the ASA group and the blank group (FIG. 5).

| Tested Solution | Onset time (min) | Duration (min) |
|---|---|---|
| B | 15 to 30 after injection | >60 |
| S | 15 to 30 after injection | <45 |
| Blank | ineffective | null |

Description of the experimental results: The solution B began to affect the pain threshold of the mice 15 to 30 minutes after intraperitoneal injection, resulting in an increase of the pain threshold, and the pain threshold of the mice did not yet return to the baseline value 90 minutes after injection; the solution S began to boost the pain threshold 15 to 30 minutes after injection, but the pain threshold returned to the baseline value after about 60 minutes. For most of the time after the solution B began to boost the pain threshold, the thresholds of the mice in the B group were higher than those of the mice, boosted by the solution S, in the S group.

Therefore, the sodium salt solution of the orthovanadate/citrate complex has an analgesic effect. Not only can orthovanadate/citrate complex exert an analgesic effect with intensities similar to those of ASA at concentrations far lower than that of the ASA solution, but it has a significantly longer duration of action (>60 min) than that (<45 min) of ASA.

### Example 8: Analgesic Experiment of Sodium Salt Solution of Metavanadate/Citrate Complex

The solution E was injected at the dose standard of 0.97 mg/kg (based on the atomic mass of vanadium), and collected data were compared with those of the ASA group and the blank group (FIG. 6).

| Tested Solution | Onset time (min) | Duration (min) |
|---|---|---|
| E | 15 to 30 after injection | >60 |
| S | 15 to 30 after injection | <45 |
| Blank | ineffective | null |

Description of the experimental results: The solution E began to affect the pain threshold of the mice 15 to 30 minutes after intraperitoneal injection, resulting in an increase of the pain threshold, and the pain threshold of the mice did not yet return to a baseline value 90 minutes after injection; the solution S began to boost the pain threshold 15 to 30 minutes after injection, but the pain threshold returned to the baseline value after about 60 minutes. For most of the time after the solution E began to boost the pain threshold, the thresholds of the mice in the E group were higher than those of the mice, boosted by the solution S, in the S group.

Therefore, the sodium salt solution of the metavanadate/citrate complex has an analgesic effect. Not only can metavanadate/citrate complex exert an analgesic effect with intensities similar to those of ASA at concentrations far lower than that of the ASA solution, but it has a significantly longer duration of action (>60 min) than that (<45 min) of ASA.

### Example 9: Analgesic Experiment of Potassium Salt Solution of Metavanadate/Citrate Complex

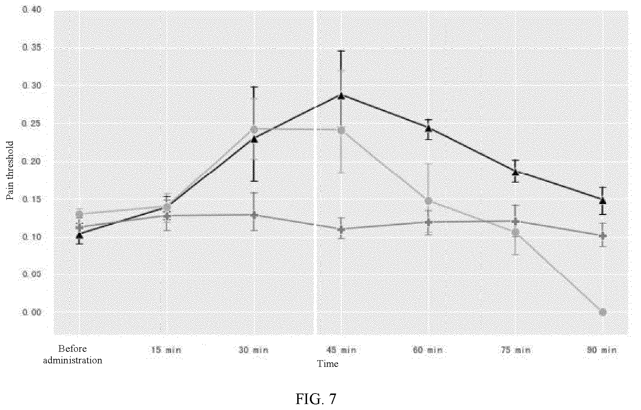

The solution F was injected at the dose standard of 0.97 mg/kg (based on the atomic mass of vanadium), and collected data were compared with those of the ASA group and the blank group (FIG. 7).

| Tested Solution | Onset time (min) | Duration (min) |
|---|---|---|
| F | 15 to 30 after injection | >60 |
| S | 15 to 30 after injection | <45 |
| Blank | ineffective | null |

Description of the experimental results: The solution F began to affect the pain threshold of the mice 15 to 30 minutes after intraperitoneal injection, resulting in an increase of the pain threshold, and the pain threshold of the mice did not yet return to a baseline value 90 minutes after injection; the solution S began to boost the pain threshold 15 to 30 minutes after injection, but the pain threshold returned to the baseline value after about 60 minutes. For most of the time after the solution F began to boost the pain threshold, the thresholds of the mice in the F group were higher than those of the mice, boosted by the solution S, in the S group.

Therefore, the potassium salt solution of the metavanadate/citrate complex has an analgesic effect. Not only can metavanadate/citrate complex exert an analgesic with intensities similar to those of ASA at concentrations far lower than that of the ASA solution, but it has a significantly longer duration of action (>60 min) than that (<45 min) of ASA.

Explanation: Environmental factors, individual difference of mice and nuance in testing may all have certain impact on the absolute values of analgesic intensity and duration of action, but in comparison to the aspirin control group, steady trend has been clearly demonstrated in all groups of A, B, C, D, E and F: a) the effective concentration of any of the tested vanadium compounds (i.e. the concentration producing an analgesic effect) was much lower than that of aspirin; b) the analgesic intensity is similar to or higher than that of aspirin; and c) the effective duration is longer than that of aspirin.

### Example 10: Analgesic Experiment of Vanadyl Sulfate

The solution G1 was injected through the tail vein at the dose standard of 0.49 mg/kg (based on the atomic mass of vanadium), and collected data were compared with those of the ASA group and the blank group.

| Tested Solution | Onset time (min) | Duration (min) |
|---|---|---|
| G1 | 0 to 15 after injection | >60 |
| S | 0 to 15 after injection | <45 |
| Blank | ineffective | null |

### Example 13: Analgesic Experiment of Bis(acetylacetonato)oxovanadium

The solution K was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| K | 15 to 30 after injection | >60 |
| S | 0 to 15 after injection | <45 |
| Blank | ineffective | null |

### Example 14: Analgesic Experiment of Vanadyl Oxalate

The solution L was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| L | 15 to 30 after injection | >60 |
| S | 15 to 30 after injection | <45 |
| Blank | ineffective | null |

### Example 15: Analgesic Experiment of Vanadyl Picolinate

The solution M was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| M | 15 to 30 after injection | >60 |
| S | 15 to 30 after injection | <45 |
| Blank | ineffective | null |

### Example 16: Analgesic Experiment of Sodium Salt Solution of Orthovanadate/Ethylene Glycol Complex

The solution N was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| N | 15 to 30 after injection | >60 |
| S | 15 to 30 after injection | <45 |
| Blank | ineffective | null |

### Example 17: Analgesic Experiment of Sodium Salt Solution of Orthovanadate/Propylene Glycol Complex

The solution O was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| O | 15 to 30 after injection | >60 |
| S | 15 to 30 after injection | <45 |
| Blank | ineffective | null |

### Example 18: Analgesic Experiment of Sodium Salt Solution of Orthovanadate/Glycerol Complex

The solution P was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| P | 15 to 30 after injection | >60 |
| S | 15 to 30 after injection | <45 |
| Blank | ineffective | null |

### Example 19: Analgesic Experiment of Sodium Salt Solution of Orthovanadate/Lactate Complex

The solution Q was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| Q | 15 to 30 after injection | >60 |
| S | 15 to 30 after injection | <45 |
| Blank | ineffective | null |

### Example 20: Analgesic Experiment of Sodium Salt Solution of Orthovanadate/Glycolate Complex

The solution R was intraperitoneally injected at the dose standard of about 0.97 mg/kg (based on the atomic mass of vanadium), and collected data were compared with those of the ASA group and the blank group.

| Tested Solution | Onset time (min) | Duration (min) |
|---|---|---|
| R | 15 to 30 after injection | >60 |
| S | 15 to 30 after injection | <45 |
| Blank | ineffective | null |

### Example 21: Effective Concentration and Plateau Experiment of Vanadium Compounds

Since the various vanadium (IV) or vanadium (V) compounds (such as the inorganic salts and the vanadium complexes containing organic ligands) have analgesic effects, it can be reckoned that the possibility of the drug ligands to participate in pain suppression is low, and their only role is being carriers for the vanadium element: the vanadium compounds may dissociate after entering the body and before reaching a (possibly more than one) target of analgesia, that is, after entering the body the vanadium element may be dissociated from the original ligands, and vanadium element is re-complexed with the natural ligands contained in the body (such as blood) instead, particularly such inorganic ligands as chloride ions, phosphate ions or hydrogen phosphate ions, and organic ligands containing O-, N- and S-donors. And then analgesic reactions would take place as the next step. In addition, the tetravalent or pentavalent vanadium compounds can rapidly create new equilibrium through oxidation-reduction reaction under physiological conditions. Based on this consideration, the tail vein injection of inorganic salts of vanadium (VOSO₄ and Na₃VO₄) not only allows the vanadium element to enter the blood directly, but it can rapidly bind to the natural ligands in the body to form an effective form required by analgesia. Compared with the vanadium compounds containing organic ligands (particularly tightly bound organic vanadium complexes), VOSO₄ and Na₃VO₄ are equivalent to "naked" donors of the vanadium element (referred to herein as "naked vanadium", the former provides tetravalent vanadium (such as VO²⁺), while the latter provides pentavalent vanadium (such as VO₄³⁻, HVO₄²⁻, H₂VO₄⁻ or VO³⁺), omitting the step of dissociation from their own ligands and also avoiding the risk of being expelled out of the body without being complexed with the natural ligands.

The relationship between the "naked vanadium" and the actual active analgesic substances is the closest, and by administering different doses of the "naked vanadium" donor VOSO₄ or Na₃VO₄, the inventor found the minimum concentrations of tetravalent vanadium and pentavalent vanadium in blood that would have an effect on mice. The minimum effective dose, i.e. about 1.0 × 10⁻³ mg/kg, was found first (see "Effective concentration data table"), from which the minimum effective vanadium concentration in the blood of mice can be calculated using following calculation method:
Assuming that an average blood volume of mice is 2 mL/30 g, the concentration in blood after tail vein injection at a dose of 1.0 × 10⁻³ mg/kg is: (1.0 × 10⁻⁶ mg/g × 30 g) / (51 × 2) = 2.9 × 10⁻⁷ M (or 0.29 µM).

Therefore, the minimum concentration of the vanadium compound in blood to produce an analgesic effect on mice is about 0.29 µM. If the conversion relationship between the doses administered to human and experimental animals is 1:9.3, it can be concluded that the minimum concentration of the vanadium compound in blood to produce an analgesic effect on the human body is 0.031 µM.

Moreover, the inventor found that as the dose of vanadium increases, both the intensity and the duration of action increase as well. However, when the concentration of vanadium in blood reaches a certain level, the analgesic effect will reach a plateau, i.e., the stage at which analgesic intensity will not increase or even decrease and/or the duration of action will not become longer (or become irregularly unpredictable) with the increase of the doses.

When the VOSO₄ or Na₃VO₄ solution was injected through the tail vein, both the plateau doses were ≥0.49 mg/kg (see "Plateau experiment data table"), equivalent to the blood concentration of 140 µM in mice. If the conversion relationship between the doses administered to human and experimental animals is 1:9.3, it can be concluded that when the concentration of vanadium reaches 15 µM in human body, the analgesic plateau will appear.

It can be predicted that the minimum effective doses of the other vanadium compounds are associated with the plateau doses as well as the affinity of between vanadium and its ligand in a complex. Taking BMOV as an example, the dose of BMOV reaching the plateau is about ≥0.97 mg/kg.

**Plateau experiment data table:**

| Tested substance/ solution | | Dose injected through tail vein (based on the atomic mass of vanadium, unit: mg/kg) | Maximum analgesic intensity (mean, unit: g) | Duration (unit: min) | Number of samples |
|---|---|---|---|---|---|
| A | A5 | 0.25 | ~0.45 | >60 | 6 |
| | A1 | 0.49 | ~0.55 | >60 | 6 |
| | A4 | 0.97 | ~0.30 | <60 | 6 |
| G | G3 | 0.25 | ~0.46 | >60 | 6 |
| | G1 | 0.49 | ~0.49 | >60 | 6 |
| | G2 | 0.97 | ~0.49 | no obvious difference from the dose of 0.49 mg/kg | 6 |

**Effective concentration data table**

| Tested substance/ solution | | Dose injected through tail vein (unit: mg/kg) | Onset time (unit: min) | Duration (unit: min) | Number of samples |
|---|---|---|---|---|---|
| A | A6 | 8.2 × 10⁻⁴ | 0-15 | <60 | 6 |
| G | G4 | 8.2 × 10⁻⁴ | 0-15 | <60 | 6 |

## Claims

1. A positive-tetravalent vanadium compound or a positive-pentavalent vanadium compound comprising a vanadium-oxygen bond for use in treating pain, wherein the positive-tetravalent vanadium compound or the positive-pentavalent vanadium compound is used in an amount of 0.00001 mg/kg body weight to 5 mg/kg body weight, based on the mass of vanadium, and the compound is prepared into an injection administered subcutaneously, intramuscularly or intravenously, with the proviso that the positive-tetravalent vanadium compound is not a metal complex formed by VO(IV) and a ligand of alpha-hydroxypyranone or alpha-hydroxypyridone type.

2. The compound for the use of claim 1, wherein the positive-tetravalent vanadium compound is selected from the group consisting of an inorganic salt, an inorganic acid salt, an organic acid salt, and a complex formed thereby together with an organic ligand or ligands.

3. The compound for the use of claim 2, wherein the positive-tetravalent vanadium compound is selected from the group consisting of VCl₄, VOCl₂, VOSO₄, (VO)₃(PO₄)₂, VOHPO₄, and VO(H₂PO₄)₂.

4. The compound for the use of claim 1, wherein the pentavalent vanadium compound is an alkali metal salt, an alkaline earth metal salt or a transition metal salt of orthovanadic acid or metavanadic acid.

5. The compound for the use of claim 4, wherein the pentavalent vanadium compound is sodium orthovanadate or potassium orthovanadate.

6. The compound for the use of claim 4, wherein the pentavalent vanadium compound is sodium metavanadate or potassium metavanadate.

7. The compound for the use of claim 1, wherein the tetravalent vanadium compound is a compound formed between a tetravalent vanadium moiety and a polycarboxylic acid or an organic ligand or ligands, or the pentavalent vanadium compound is a compound formed between a pentavalent vanadium moiety and a polycarboxylic acid or an organic ligand or ligands.

8. The compound for the use of claim 7, wherein the compound is formed between a pentavalent vanadium moiety and citric acid.

9. The compound for the use of claim 1, wherein the compound is selected from the group consisting of lithium orthovanadate, sodium orthovanadate, potassium orthovanadate, lithium polyvanadate, sodium polyvanadate, potassium polyvanadate, lithium metavanadate, sodium metavanadate, potassium metavanadate, a quaternary ammonium salt of metavanadate, vanadyl sulfate, vanadyl dichloride.

10. The compound for the use of claim 1, wherein the compound is selected from the group consisting of vanadyl sulfate, sodium orthovanadate, potassium orthovanadate, sodium metavanadate, potassium metavanadate, and their vanadium compound prepared with citric acid, tartaric acid, lactic acid, glycolic acid, ethylene glycol, glycerol, or triethanolamine [tris(2-hydroxyethyl)amine] at a molar ratio of 1:0.5 to 1:1000, and a citrate complex K[VO₂(C₆H₆O₇)]·H₂O.

11. The compound for the use of any one of claims 1 to 8, wherein the compound is administered intravenously, or subcutaneously.

## Patentansprüche

1. Eine positiv-vierwertige Vanadiumverbindung oder eine positiv-fünfwertige Vanadiumverbindung, die eine Vanadium-Sauerstoff-Bindung beinhaltet, zur Verwendung bei der Behandlung von Schmerzen, wobei die positiv-vierwertige Vanadiumverbindung oder die positiv-fünfwertige Vanadiumverbindung in einer Menge von 0,00001 mg/kg Körpergewicht bis 5 mg/kg Körpergewicht, bezogen auf die Masse von Vanadium, verwendet wird und die Verbindung zur Injektion hergestellt wird, die subkutan, intramuskulär oder intravenös verabreicht wird, mit der Maßgabe, dass die positiv-vierwertige Vanadiumverbindung kein Metallkomplex ist, der durch VO(IV) und einen Liganden vom alpha-Hydroxypyranon- oder alpha-Hydroxypyridon-Typ gebildet wird.

2. Verbindung zur Verwendung gemäß Anspruch 1, wobei die positiv-vierwertige Vanadiumverbindung aus der Gruppe ausgewählt ist, die aus einem anorganischen Salz, einem anorganischen Hydrogensalz, einem organischen Hydrogensalz und einem Komplex, der daraus zusammen mit einem oder mehreren organischen Liganden gebildet wird, besteht.

3. Verbindung zur Verwendung gemäß Anspruch 2, wobei die positiv-vierwertige Vanadiumverbindung aus der Gruppe ausgewählt ist, die aus VCl₄, VOCl₂, VOSO₄, (VO)₃(PO₄)₂, VOHPO₄ und VO(H₂PO₄)₂ besteht.

4. Verbindung zur Verwendung gemäß Anspruch 1, wobei die fünfwertige Vanadiumverbindung ein Alkalimetallsalz, ein Erdalkalimetallsalz oder ein Übergangsmetallsalz von Orthovanadinsäure oder Metavanadinsäure ist.

5. Verbindung zur Verwendung gemäß Anspruch 4, wobei die fünfwertige Vanadiumverbindung Natriumorthovanadat oder Kaliumorthovanadat ist.

6. Verbindung zur Verwendung gemäß Anspruch 4, wobei die fünfwertige Vanadiumverbindung Natriummetavanadat oder Kaliummetavanadat ist.

7. Verbindung zur Verwendung gemäß Anspruch 1, wobei die vierwertige Vanadiumverbindung eine Verbindung ist, die zwischen einem vierwertigen Vanadiummolekülteil und einer Polycarbonsäure oder einem oder mehreren organischen Liganden gebildet wird, oder die fünfwertige Vanadiumverbindung eine Verbindung ist, die zwischen einem fünfwertigen Vanadiummolekülteil und einer Polycarbonsäure oder einem oder mehreren organischen Liganden gebildet wird.

8. Verbindung zur Verwendung gemäß Anspruch 7, wobei die Verbindung zwischen einem fünfwertigen Vanadiummolekülteil und Zitronensäure gebildet wird.

9. Verbindung zur Verwendung gemäß Anspruch 1, wobei die Verbindung aus der Gruppe ausgewählt ist, die aus Lithiumorthovanadat, Natriumorthovanadat, Kaliumorthovanadat, Lithiumpolyvanadat, Natriumpolyvanadat, Kaliumpolyvanadat, Lithiummetavanadat, Natriummetavanadat, Kaliummetavanadat, einem quaternären Ammoniumsalz von Metavanadat, Vanadylsulfat, Vanadyldichlorid besteht.

10. Verbindung zur Verwendung gemäß Anspruch 1, wobei die Verbindung aus der Gruppe ausgewählt ist, die aus Vanadylsulfat, Natriumorthovanadat, Kaliumorthovanadat, Natriummetavanadat, Kaliummetavanadat und ihrer Vanadiumverbindung, die mit Zitronensäure, Weinsäure, Milchsäure, Glykolsäure, Ethylenglykol, Glycerin oder Triethanolamin [Tris(2-hydroxyethyl)amin] in einem Molverhältnis von 1 : 0,5 bis 1 : 1000 hergestellt wird, und einem Citratkomplex K[VO₂(C₆H₆O₇)]·H₂O besteht.

11. Verbindung zur Verwendung gemäß einem der Ansprüche 1 bis 8, wobei die Verbindung intravenös oder subkutan verabreicht wird.

## Revendications

1. Un composé de vanadium tétravalent positif ou un composé de vanadium pentavalent positif comprenant une liaison vanadium-oxygène pour son utilisation dans le traitement de la douleur, dans lequel le composé de vanadium tétravalent positif ou le composé de vanadium pentavalent positif est utilisé en une quantité allant de 0,00001 mg/kg de poids corporel à 5 mg/kg de poids corporel, sur la base de la masse de vanadium, et le composé est préparé en une injection administrée par voie sous-cutanée, intramusculaire ou intraveineuse, à condition que le composé de vanadium tétravalent positif ne soit pas un complexe métallique formé par VO(IV) et un ligand de type alpha-hydroxypyranone ou alpha-hydroxypyridone.

2. Le composé pour son utilisation selon la revendication 1, dans lequel le composé de vanadium tétravalent positif est choisi dans le groupe constitué d'un sel inorganique, d'un sel d'acide inorganique, d'un sel d'acide organique, et d'un complexe formé par ceux-ci conjointement avec un ou des ligands organiques.

3. Le composé pour son utilisation selon la revendication 2, dans lequel le composé de vanadium tétravalent positif est choisi dans le groupe constitué de VCl₄, VOCl₂, VOSO₄, (VO)₃(PO₄)₂, VOHPO₄, et VO(H₂PO₄)₂.

4. Le composé pour son utilisation selon la revendication 1, dans lequel le composé de vanadium pentavalent est un sel de métal alcalin, un sel de métal alcalino-terreux ou un sel de métal de transition d'acide orthovanadique ou d'acide métavanadique.

5. Le composé pour son utilisation selon la revendication 4, dans lequel le composé de vanadium pentavalent est l'orthovanadate de sodium ou l'orthovanadate de potassium.

6. Le composé pour son utilisation selon la revendication 4, dans lequel le composé de vanadium pentavalent est le métavanadate de sodium ou le métavanadate de potassium.

7. Le composé pour son utilisation selon la revendication 1, dans lequel le composé de vanadium tétravalent est un composé formé entre une partie de vanadium tétravalent et un acide polycarboxylique ou un ou des ligands organiques, ou le composé de vanadium pentavalent est un composé formé entre une partie de vanadium pentavalent et un acide polycarboxylique ou un ou des ligands organiques.

8. Le composé pour son utilisation selon la revendication 7, dans lequel le composé est formé entre une partie de vanadium pentavalent et de l'acide citrique.

9. Le composé pour son utilisation selon la revendication 1, dans lequel le composé est choisi dans le groupe constitué de l'orthovanadate de lithium, de l'orthovanadate de sodium, de l'orthovanadate de potassium, du polyvanadate de lithium, du polyvanadate de sodium, du polyvanadate de potassium, du métavanadate de lithium, du métavanadate de sodium, du métavanadate de potassium, d'un sel d'ammonium quaternaire de métavanadate, du sulfate de vanadyle, du dichlorure de vanadyle.

10. Le composé pour son utilisation selon la revendication 1, dans lequel le composé est choisi dans le groupe constitué du sulfate de vanadyle, de l'orthovanadate de sodium, de l'orthovanadate de potassium, du métavanadate de sodium, du métavanadate de potassium, et de leur composé de vanadium préparé avec de l'acide citrique, de l'acide tartrique, de l'acide lactique, de l'acide glycolique, de l'éthylène glycol, du glycérol, ou de la triéthanolamine [tris(2-hydroxyéthyl)amine] à un rapport molaire allant de 1/0,5 à 1/1000, et d'un complexe de citrate K[VO₂(C₆H₆O₇)]·H₂O.

11. Le composé pour son utilisation selon l'une quelconque des revendications 1 à 8, dans lequel le composé est administré par voie intraveineuse, ou sous-cutanée.
